# EUROPEAN PATENT SPECIFICATION

(11) **EP 2 928 563 B1**
(45) Date of publication and mention of the grant of the patent: **11.04.2018**
(21) Application number: 13812289.0
(22) Date of filing: 03.12.2013
(51) Int. Cl.: A61Q 1/04, A61K 8/58, A61Q 5/12, A61K 8/89, A61Q 5/06, A61Q 19/00, A61K 8/891, A61K 8/892, A61K 8/06

(54) **COMPOSITION COMPRISING A SILICONE RESIN AND A SILICONE GUM, PERSONAL CARE PRODUCTS CONTAINING THE SAME**
ZUSAMMENSETZUNG MIT EINEM SILIKONHARZ UND SILIKONGUMMI, KÖRPERPFLEGEPRODUKTE DAMIT
COMPOSITION COMPRENANT UNE RÉSINE DE SILICONE ET UNE GOMME DE SILICONE, PRODUITS DE SOIN PERSONNEL LA CONTENANT

(30) Priority: 04.12.2012 US 201213693474
(43) Date of publication of application: 14.10.2015
(73) Proprietor: Momentive Performance Materials Inc., Waterford, NY 12188 (US)
(72) Inventor: DUSSAUD, Anne, Tarrytown, NY 10591 (US); RANA, Bhavna, White Plains, NY 10607 (US); SPERRING, Susan, Pomona, NY 10970 (US)
(74) Representative: Zinnecker, Armin
(86) International application number: PCT/US2013/072824
(87) International publication number: WO 2014/089044

(56) References cited:
- WO-A1-02/34221
- WO-A1-02/089760
- WO-A1-03/026599
- WO-A2-02/03949
- FR-A1- 2 688 134
- US-A- 3 669 706
- US-A- 4 906 459
- US-A- 5 246 694
- US-A- 6 071 503
- US-A1- 2006 045 893
- US-A1- 2009 004 132
- "VelvesilÂ TM FX gel-powder", IP.COM JOURNAL, IP.COM INC., WEST HENRIETTA, NY, US, 16 April 2008 (2008-04-16), XP013124596, ISSN: 1533-0001

## Description

### FIELD OF THE INVENTION

This invention generally relates to silicone compositions and personal care products containing same. More particularly, the invention relates to silicone compositions containing blends of silicone resin(s) and silicone gum(s) and to personal care products providing shine or gloss, e.g., hair conditioners, hair sprays, hair gels, hair creams, lip glosses, and the like, formulated with such compositions.

### BACKGROUND OF THE INVENTION

The use of linear silicones of high molecular weight has been known for a long time to provide hair conditioning and increase hair luster. However, linear silicones have a tendency to flow and do not provide acceptably stable films on hair fibers. In particular, it is often found that at high dosages of silicone gum, hair fibers tend to stick to each other and the hair takes on a greasy appearance.

It is well known that MQ silicone resin and linear silicone blends form a structured network which is widely used in silicone pressure sensitive adhesives. Such blends are also commonly used for transfer resistance of pigmented formulations in cosmetics, e.g., lipsticks, foundations, and the like. However, in these applications, the blends of silicone resin and linear silicones are tacky and do not provide the clean, non-tacky and smooth finish that would be required of an acceptable hair care product.
Relevant prior art comprises the following documents: US 6,071,503 A, US 4,906,459 A, US 2006/045893 A1, WO 02/089760 A1, WO 02/03949 A2, WO 03/026599 A1, FR 2 688 134 A1 and WO 02/34221 A1 disclose cosmetic compositions comprising MQ silicone resins. US 5,246,694 A discloses shampooing compositions comprising silesquioxane resins or MQ resins. US 3,669,706 A discloses a mixture comprising a silicone elastomer. US 2009/004132 A1 discloses cosmetic compositions comprising a blend of silicone gum and T-resins. The article "VelvesilÂ TM FX gel-powder", ip.com Journal, West Henrietta, NY, US, (2008-04-16), ISSN 1533-0001 also discloses a cosmetic composition comprising an MQ silicone resin.

### SUMMARY OF THE INVENTION

In accordance with the present invention, there is provided a composition which comprises:
a) an MT silicone resin; and,
b) silicone gum,
the mixture of silicone resin (a) and silicone gum (b) having a softening point of 50°C or greater and an elastic modulus at ambient temperature of 10⁶Pa or less.

Surprisingly, it has been found that the foregoing composition forms stable films on hair fibers and significantly reduces the problem of excessive stickiness. When applied to hair, formulations containing the composition of this invention provide high luster while leaving the hair with a smooth feel and with little if any fiber clumping.

### DESCRIPTION OF THE INVENTION

MT silicone resin (a) of the composition herein contains one or more M units, represented by the formula R¹₃SiO_{1/3}, and T units represented by the formula R³SiO_{3/2} wherein each R¹ and R³ is independently a hydroxyl radical or monovalent hydrocarbon radical.

Suitable monovalent hydrocarbon radicals R¹ and R³ include acyclic
hydrocarbon radicals, alicyclic hydrocarbon radicals and aromatic hydrocarbon radicals. Preferred monovalent hydrocarbon radicals are alkyl radicals, aryl radicals and aralkyl radicals.

As used herein, the expression "acyclic hydrocarbon radical" means a straight or branched chain hydrocarbon radical, preferably containing up 22 carbon atoms, which may be saturated or unsaturated and which may contain one or more hetero atoms, e.g., oxygen, nitrogen, etc., and/or one or more functional groups and/or atoms, e.g., hydroxyl, halo, especially chloro and fluoro, and the like, in substitution of a like number of hydrocarbyl hydrogen atoms.

Suitable monovalent acyclic hydrocarbon radicals include, for example, alkyl, alkenyl, alkynyl, hydroxyalkyl, cyanoalkyl, carboxyalkyl, alkyloxy, oxaalkyl, alkylcarbonyloxaalkylene, carboxamide and haloalkyl, such as, for example, methyl, ethyl, sec-butyl, tert-butyl, octyl, decyl, dodecyl, cetyl, stearyl, ethenyl, propenyl, butynyl, hydroxypropyl, cyanoethyl, butoxy, 23,8-trioxadecanyl, carboxymethyl, chloromethyl, trifluoromethyl, and 3,3,3-trifluoropropyl.

As used herein, the expression "alicyclic hydrocarbon radical" means a radical containing one or more saturated hydrocarbon rings, preferably containing from 4 to 12 carbon atoms per ring, which may optionally be substituted on one or more of the rings with one or more alkyl radicals, each preferably containing from 2 to 6 carbon atoms per alkyl radical, halo radicals or other functional groups and which, in the case of a monovalent alicyclic hydrocarbon radical containing two or more rings, may be fused rings. Suitable monovalent alicyclic hydrocarbon radicals include, for example, cyclohexyl and cyclooctyl.

As used herein, the expression "aromatic hydrocarbon radical" means a hydrocarbon radical containing one or more aromatic rings per radical which may optionally, be substituted on the aromatic rings with one or more alkyl radicals, each preferably containing from 2 to 6 carbon atoms per alkyl radical, halo radicals or other functional groups and which, in the case of a monovalent aromatic hydrocarbon radical containing two or more rings, may be fused rings. Suitable monovalent aromatic hydrocarbon radicals include, for example, phenyl, tolyl, 2,4,6-trimethylphenyl, 1,2-isopropylmethylphenyl, 1-pentalenyl, naphthyl, anthryl. As used herein, the term "aralkyl" means an aromatic derivative of an alkyl group, preferably a (C₂-C₆)alkyl group, wherein the alkyl portion of the aromatic derivative may optionally, be interrupted by an oxygen atom such as, for example, phenylethyl, phenylpropyl, 2-(1-naphthyl)ethyl, preferably phenylpropyl, phenyoxypropyl, biphenyloxypropyl, and the like.

In a preferred embodiment, silicone resin (a) contains 30 percent or greater, preferably 40 percent or greater, and more preferably 50 percent or greater, T units.

The ratio of M to T units ranges from 1:1 to 1:7 and the softening point of the resin ranges from 50° to 110°C. Useful silicone resins of this type are disclosed in U.S. 2011/0040062.

Silicone resin (a) has a number average molecular weight of 10,000 or greater, preferably up to 1,000,000, and more preferably from 10,000 to 500,000.

In a preferred embodiment, silicone gum (b) of the composition herein is selected from those silicones having a viscosity of from 0.3 - 200,000 and preferably from 750 - 2,000 Pa.s (from 300 to 200,000,000, and preferably from 750,000 to 2,000,000, centipoise (cps)) at 25°C. The viscosity of silicone gum (b) can be readily measured employing known and conventional viscosity measurement apparatus and techniques.

In a preferred embodiment, silicone gum (b) exhibiting the foregoing viscosity characteristic contains M' units, represented by the formula R¹₃SiO_{1/2}, and one or more additional units selected from among D' units, represented by the formula R⁵₂SiO_{2/2}, T' units, represented by the formula R⁶SiO_{3/2}, and Q' units, represented by the formula SiO_{4/2}, and mixtures thereof, wherein each R⁴, R⁵ and R⁶ is independently a hydroxyl radical or a monovalent hydrocarbon radical.

Suitable monovalent hydrocarbon radicals R⁴, R⁵ and R⁶ include acyclic hydrocarbon radicals, alicyclic hydrocarbon radicals and aromatic hydrocarbon radicals as defined and exemplified, *supra.*

Suitable silicone gums (b) are known and are commercially available. For example, the gums can be prepared according to the method disclosed in U.S. Patent No. 2,814,601, wherein an appropriate siloxane is reacted with an aqueous acid in a closed system until the viscosity of the siloxane has become essentially constant. The product is then washed free of acid. Specific examples of useful silicone gums (b), all from Momentive Performance Materials Inc., include Silsoft 1215 (dimethiconol gum in cyclodimethicone solvent D5, SE30 (dimethicone gum), Viscasil 60M (polydimethysiloxane gum) and Silsoft AX (alkyl-modified aminosilicone).

As for many products applications including hair care products, it is preferred that all, or nearly all, of the mixtures of silicone resin (a) and silicone gum (b) are non-crosslinked, the invention provides for a non-crosslinked mixture of resin (a) and gum (b) that contains little or no crosslinkable functionality, e.g., R¹ and R³ of resin (a) and R⁴, R⁵ and R⁶ of gum (b) will contain no more than a few crosslinkable groups, and/or crosslinking conditions will be avoided in the preparation of the mixtures of these silicones and their use in manufacturing the desired personal care product

The mixture of silicone resin (a) and silicone gum (b) herein must have a softening point of 50°C or greater, preferably 60°C or greater and more preferably 70°C or greater, and an elastic modulus at ambient temperature of 10⁶ Pa or less, preferably 5x10⁵ Pa or less and more preferably 3x10⁵ Pa or less. In general, these characteristics of softening point and elastic modulus can be obtained with mixtures of silicone resin(s) (a) and silicone gum(s) (b) with weight ratios of resin(s) (a) to gum(s) (b) ranging from 0.4 to 6, preferably ranging from 0.5 to 5 and more preferably from 0.7 to 4.

Since the mixtures of silicone resin (a) and silicone gum (b) of this invention have softening points of 50°C or greater, which is to say, they are solids at ambient temperature, it may be advantageous for formulating a particular personal care product to dilute the mixture of silicone resin(s) (a) and silicone gum(s) (b) with one or more volatile organic solvents (c), e.g., an organosilicon-containing solvent such a ethyl trisiloxane, octyl trisiloxane, cyclodimethicone, and the like, and/or one or more other type organic solvents such as the volatile paraffinic solvents and the aromatic hydrocarbon solvents. Examples of such solvents include the C₅-C₁₂ acyclic and cyclic alkanes, e.g., the straight chain and isomeric pentanes, hexanes, heptanes, octanes, nonanes, decanes, undecanes, dodecanes, etc., and their cyclic analogs, and aromatic solvents as, for example, exemplified by benzene, toulene, the xylenes, mesitylene, and the like. Where utilized, volatile organic solvents) (c) can generally be combined with mixtures of resin(s) (a) and gum(s) (b) in a weight ratio of solvent to resin/gum mixture from 200 to 0.1, preferably from 100 to 1 and more preferably from 60 to 5.

In a preferred embodiment, an organic solvent solution of resin (a) and gum (b) can be formulated as an aqueous or non-aqueous spray, an aqueous or non-aqueous foam or mousse, a water-in-oil (w/o) emulsion or oil-in-water (o/w) emulsion employing procedures well known in the art for providing hair care products.

The personal care formulations of the invention can also contain one or more other ingredients known for use in such products in known and conventional amounts such as humectants (panthenol, butylene glycol, sorbitol, glycerin, other polyols), amino acids, natural moisturizing factors (sodium PCA), nonionic waxes (fatty alcohols, ethoxylated waxes, glycerol stearate, bee waxes, paraffin waxes etc.), esters, triglyceride oils (olive oil, jojoba oil, sunflower oil, coconut oil, argan oil, grapeseed oil etc..), natural butters (shea butter, cocoa butter), emulsifiers (silicone emulsifiers, silicone polyether copolymers, organic emulsifiers), anionic or amphoteric surfactants (cocobetaine, SLES, isothionate, sugar surfactants), spreading agents such as silicone superspreaders, solid particulates, pigments, minerals (talc, micas, iron oxides, boron nitride, titanium oxide, clays), permanent and semi-petimanent hair dyes, fragrances, actives such as plant extracts, polyphenols, polysaccharides (chitosan), proteins (keratin, silk protein, wheat proteins), lipids, sterols, antidandruff actives, salicylic acid, glycolic acid, hair growth actives, anti-aging actives (retinol, alpha-hydroxy acids), niacinamide, reducing agents (thioglycolates, cysteamine), sulfites, oxidizing agents (hydrogen peroxide, bromates), relaxers (sodium hydroxides, guanidines), crosslinking agents (aldehydes, epoxy containing compounds, silanes, enzymes), styling polymers (PVP, acrylate copolymers), thickening polymers (acrylates, polyacrylamide, cellulose, starch, polysaccharide gums, pectins, etc.), deposition aid polymers (cationic guars, cationic cellulose, merquats), preservatives, biocides (phenoxyethanol, potassium sorbate, benzoic acid, sorbic acids, etc.), antioxidants (vitamin E), UVA UVB sunscreens, sunless tanning agents (dihydroxyacetone), and so forth.

### EXAMPLES A-F, EXAMPLES 1-7

### (1) Silicone Resins (a) and Silicone Gums (b)

| | | |
|---|---|---|
| Silicone Resin | Silform Flex | MT Resin |
| Silicone Resin | SRI 000 | MQ Resin |
| Linear Silicone in Volatile Silicone D5 | Silsoft 1215 | Dimethiconol Gum |
| Linear Silicone | SE30 | Dimethicone Gum |
| Linear Silicone | Viscasil 60M | Polydimethylsiloxane Gum |
| Linear Silicone | Silsoft AX | Alkyl-modified Aminosilicone |

Silform Flex MT resin and SR1000 MQ resin are available from Momentive Performance Materials Inc. Silsoft 1215 contains 15% by weight of dimethiconol gum in silicone solvent D5 and is available from Momentive Performance Materials Inc. SE30 is a dimethicone gum also available from Momentive Performance Materials Inc. Viscasil 60 M is a polydimethylsiloxane gum having a viscosity of about 60 Pa.s (60,000 cps) and is available from Momentive Performance Materials Inc.

### (2) Test Methods

### a. Softening Point and Elastic Modulus

Mixtures of resin (a) and gum (b) with different weight ratios of resin (a) to gum (b) were dissolved in cyclodimethicone solvent D5, placed in a flat aluminium pan and dried at 90°C until the D5 had completely evaporated. Elastic modulus (G') and storage modulus (G") were measured as functions of the temperature in the range of from -150°C to 150°C employing Dynamic Mechanical Analysis of TA Instrument (New Castle, DE), at a frequency of about 1 Hz. The temperature where the maximum of tan delta (ratio of G"/G') was observed was defined as the softening temperature. This method is a well established method for characterizing silicone pressure sensitive adhesives.

### b. Film Tack

60 micron films were produced in aluminium pans by drying mixtures of resin (a) and gum (b) in solvent D5. After evaporation of the D5, maximum tack forces were measured with a Stable Micro Systems Texture Analyzer (Surrey, U.K.) at a load of 100 g and a contact time of 1 s. Hair care products exhibiting a level of film tack not greater than 50g are generally considered to perform acceptably well in this regard.

### c. Hair Gloss (or Luster)

Hair luster was measured with a Murakami GoniophotoMeter (Tokyo, Japan), at an angle of incidence of 30°. Gloss factor was obtained using the total reflectance values and the formula F_{g}=(Lₘₐₓ-D)/D where Lₘₐₓ is the maximum reflectance and D is the reflectance value obtained at 0° viewing angle (diffusive reflectance). Hair luster is considered to be significantly increased when the gloss factor is higher than 1.6.

### d. Tress Volume

Fiber clumping produced a significant volume reduction of the tress. Volume reduction was measured by image analysis after the hair tress was stored 1 hour in a 90% RH humidity chamber. The tress volume was measured by counting the number of pixels of the tress area (A). A volume reduction factor was obtained using the formula Rv= 100*(Aₒ-A)-A)/(Aₒ-Aₘᵢₙ) where Aₒ was the value of the untreated tress area (very frizzy tress) and Aₘᵢₙ was the minimum tress area (ironed tress). If the volume reduction is excessive (Rᵥ>70%), the hair will appear clumpy. If the volume reduction is too low (Rᵥ<30%), the hair will appear frizzy. With a volume reduction between 40% and 60%, treated hair tress will not appear frizzy and the hair fibers will not form clumps, thus allowing the hair to flow freely.

### e. Hair Smoothness

Hair coefficient of friction µ was measured on a CSM tribometer (Needham, Ma), on a taut flat tress, with a flat stainless steel probe. Hair was considered smooth when µ < 0.12.

### (3) Hair Treatment Formulations

Examples of mixtures of resin (a) and gum (b) blends and the results of treating hair with the mixtures are presented in Tables 1A and 1B below. Examples A-F are comparative examples while examples 1 to 7 illustrate the claimed invention. The mixtures of resin (a) and gum (b) were diluted in cyclodimethicone D5 to provide a 2% by weight solids solution. A single bleached hair tress was immersed in each test solution for 1 min. Excess liquid was squeezed out and the tress was thoroughly dried to remove solvent D5 using a blow drier, the tress thereafter being placed in an oven at 45°C overnight By this procedure, approximately the same amount of silicone mixture was delivered to each hair tress sample. Tress measurements were taken after equilibration in a 50% humidity chamber.

In Tables 1A and 1B, Example A is an untreated hair tress having low luster, low tack and a rough finish. In Example B, the tress treated only with silicone gum had a very high luster but exhibited excessive clumping of its fibers resulting in the tress appearing greasy. The mixtures of resin (a) and gum (b) of Examples C and D, both of which had very low softening points, produced high tack films and excessive hair fiber clumping. The mixtures of resin (a) and gum (b) of Examples E and F, both of which had a high elastic modulus at room temperature, resulted in a hair with low luster and a rough finish.

In contrast to the compositions of comparative Examples A-F and as shown in Table 1B, the hair tresses of Examples 1-7 produced desirable properties of high gloss, low friction (smooth feel) and moderate volume reduction, i.e.,
no appreciable clumping. With a volume reduction between 40% and 60%, the hair tresses treated with the compositions of Examples 1-7 did not appear frizzy and the hair fibers avoided forming clumps as shown by the hair being able to flow freely. The silicon mixtures of Examples 1-7 therefore fulfilled all major criteria for a well-formulated and functioning hair care product whereas Examples A-E failed to meet even one of these criteria.

**Table 1A: Mixtures of Resin (a) and Gum (b)**

| Example | % MQ | % MT | % T unit in Resin | Gum (b) | Weight Ratio of Resin (a) to Gum (b) |
|---|---|---|---|---|---|
| Ex. A | | | | | |
| Ex. B | 0 | 0 | | Silsoft 1215 | |
| Ex. C | 100 | | 0 | Silsoft 1215 | 0.5 |
| Ex. D | 100 | | 0 | Silsoft 1215 | 1.3 |
| Ex. E | 100 | | 0 | Silsoft 1215 | 3.1 |
| Ex. F | 100 | | 0 | Silsoft 1215 | 4.7 |
| | | | | | |
| Ex. 1 | | 100 | 85 | Silsoft 1215 | 0.8 |
| Ex. 2 | | 100 | 85 | Silsoft 1215 | 1.3 |
| Ex. 3 | | 100 | 85 | Silsoft 1215 | 2.1 |
| Ex. 4 | | 100 | 85 | Silsoft 1215 | 3.1 |
| Ex. 5 | | 100 | 85 | Silsoft 1215 | 4.7 |
| Ex. 6 | 20 | 80 | 68 | Silsoft 1215 | 1.3 |
| Ex. 7 | 33 | 67 | 57 | Silsoft 1215 | 1.3 |

**Table 1B: Results of Hair Treatment**

| Example | Softening Point of Mixture, (°C) | G'(Pa) at 25°C | Tack (g) | Gloss Factor | Volume Reduction % | Hair Coefficient of Friction | Result(s) |
|---|---|---|---|---|---|---|---|
| Ex. A | | | 0 | 1.23 | 0 | 0.147 | low shine, frizzy hair, poor feel |
| Ex. B | -50 | 3.6x10⁴ | 19 | 2.46 | 82 | 0.084 | Clumping |
| Ex. C | 17 | 1.7x10^{b} | 113 | 2.57 | 79 | 0.101 | Clumping |
| Ex. D | 43 | 3.3x10^{b} | 161 | 2.16 | 86 | 0.100 | Clumping |
| Ex. E | 82 | 3.4x10^{b} | 0 | 1.53 | 50 | 0.146 | low shine, draggy |
| Ex. F | 82 | 2.5x10^{b} | 0 | 1.48 | - | 0.155 | low shine, draggy |
| | | | | | | | |
| Ex. 1 | 108 | 4.5x10⁴ | 30 | 2.3 | 56 | 0.093 | high shine,no,clumping,smooth |
| Ex. 2 | 106 | 445x10⁴ | 25 | 2.48 | 55 | 0.080 | high shine,no clumping, smooth |
| Ex. 3 | 94 | 1.0x10⁵ | 7 | 2.71 | 49 | 0.097 | high shine, no clumping, smooth |
| Ex. 4 | 90 | 1.8x10⁵ | 3 | 1.93 | 45 | 0.091 | high shine,no clumping, smooth |
| Ex. 5 | 85 | 2.1x10⁵ | 3 | 2.51 | - | 0.094 | high shine, noclumping, smooth |
| Ex. 6 | 92 | 7.5x10⁴ | 37 | 1.83 | - | | high shine, noclumping, smooth |
| Ex. 7 | 93 | 4.9x10⁴ | 22 | 1.62 | - | | high shine, no clumping, smooth |

### EXAMPLE G, EXAMPLE 8

It is an essential requirement of a hair care product containing a mixture of silicone resin (a) and silicone gum (b) that it be essentially devoid of cationic surfactant. This requirement is demonstrated by the comparison presented below in Table 2.

**Table 2: Results of Hair Treatment With Mixtures of Resin (a) and Gum (b) With and Without Cationic Surfactant**

| Hair Treatment | Example G | Example 8 |
|---|---|---|
| MT Resin | 10 | 10 |
| Gum SE30 | 5 | 5 |
| Cyclodimethicone D5 | 30 | 85 |
| cetrimonium chloride (cationic surfactant) | 2 | 0 |
| Water | 53 | 0 |
| ratio Resin (a)/Gum (b) | 2 | 2 |
| % T Units in Resin (a) | 85 | 85 |
| Gloss Factor | 1.5 | 2.14 |
| Tress Appearance | Clumping, Greasy | High Shine, No Clumping |

As shown in Table 2, the tress treated with a composition
(Example 8) containing no cationic surfactant exhibited a high shine with no fiber clumping thus giving the hair a clean appearance with its fibers moving freely. In contrast, the hair treated with the composition containing cationic surfactant (Example G) appeared clumpy and greasy.

### EXAMPLES 9-14

Table 3 below sets forth the composition of O/W emulsions suitable for the formulation of hair care products. Each of the illustrated compositions contains a mixture of silicone resin (a) and silicone gum (b) and no cationic surfactant.

**Table 3: O/W emulsion examples**

| Ingredient Tradename | NCI* | Description | Example 9 | Example 10 | Example 11 | Example 12 | Example 13 | Example 14 |
|---|---|---|---|---|---|---|---|---|
| AMP-95 | aminomethyl propanol | | 0.56 | | | | | 0.7 |
| Aculyn 180 | acrylates/hydroxyesters acrylates copolymer | Anionic polymer | 2 | | | | | |
| Aculyn88 | acrylates/steareth 20 methacrylate copolymer | Anionic polymer | 5 | | | | | |
| Cellosize polymer peg-10 | hydroxyethyl cellulose | nonionic polymer | | | | | | |
| Sepigel 305 | | Anionic polymer | | 5 | | | | |
| Aristof lex AVC | | Anionic polymer | | | 2 | | | |
| Carbopol 980 | | Anionic polymer | | | | | | 0.9 |
| Fixale G-100 | AMP-acrylates/allyl meth acrylates copolymers (26%) | Anionic polymer styling polymer | | | | | | 2.6 |
| Ultrez 20 | acrylates/C10-C30 alkyl acrylate copolymer | Anionic polymer | | | | 0.2 | | |
| Carbopol aqua SF-1 (30,A) | | Anionic polymer | | | | | 1 | |
| | glyceryl stearate and PEG-100stearata | nonionic surfactant | | | | 2 | 6 | |
| | cetearyl alcohol | fatty alcohol wax | | | | 1 | 3 | |
| | sodium hydroxide (18%) | | | | | 0.2 | 0.2 | |
| | panthenol | humectant | | | | 0.5 | 0.5 | |
| | dis odium EDTA | | | | | 0.05 | 0.05 | |
| Silicone MT Resin | | Silicone resin | 5 | 5 | 5 | 5 | 5 | 5 |
| Silicon Gum SE30 | | Silicone gum | 2.5 | 2.5 | 2.5 | 2.5 | 2.5 | 2.6 |
| Cyclodimethicone D5 | | Volatile silicone | 7 | 7 | | | | 7 |
| isododecane | | Volatile solvent | | | | | 7 | |
| Silsoft ETS | | volatile silicone | | 7 | 7 | 7 | | |
| wotor | | | q.s 100 | q.s 100 | q.s 100 | q.s 100 | q:s 100 | q.s 100 |

| | | | | | | | | |
|---|---|---|---|---|---|---|---|---|
| *INC=International Nomenclature of Cosmetic Ingredients | | | | | | | | |

### EXAMPLES 15 AND 16

Table 4 below sets forth the composition of WO emulsions suitable for formation of hair care products. The composition of example 17 does not fall within the scope of the invention as claimed.

**Table 4: W/O emulsion example**

| Ingredient | Description | Example 16, Wt % | Example 17, Wt % |
|---|---|---|---|
| Silform 60A | Silicone polyether copolymer | 2 | 2 |
| Isododecane | Volatile solvent | 10 | 10 |
| Silsoft ETS | Volatile silicone | 10 | 10 |
| MT resin | Silicone Resin | 5 | 5 |
| Silicone gum SE30 | Silicone Gum | 2.5 | - |
| Silsoft AX | Alkyl-modified aminosilicone | - | 1.6 |
| NaCl | | 0.8 | 0.8 |
| Butylene glycol | Humectant | 3 | 3 |
| Water | | 66.7 | 66.7 |

### EXAMPLE 17

Table 5 sets forth the formulation of a lip gloss product.

**Table 5: Lip Gloss Formulation**

| Ingredient | Wt % |
|---|---|
| MT resin | 30 |
| Viscasil 60M gum | 15 |
| Cyclodimethicone | 54.8 |
| Fragrance | 0.2 |

## Claims

1. A composition which comprises:
a) a MT resin having a molecular weight higher than 10,000 and containing one or more M units of the formula R¹₃SiO_{⅓}. and T units of the formula R³SiO_{3/2} wherein the ratio of M to T units ranges from 1:1 to 1:7, and the softening point of the MT resin ranges from 50° to 110° C wherein each R¹ and R³ is independently a hydroxyl radical or monovalent hydrocarbon radical; and,
b) silicone gum,
wherein the mixture of MT resin (a) and silicone gum (b) is non-crosslinked and has a softening point greater than 50°C and an elastic modulus at ambient temperature of less than 10⁶Pa.

2. The composition of Claim 1 wherein silicone gum (b) possesses a viscosity of from 0.3 to 200,000 Pa·s (from 300 to 200,000,000 centipoise) at 25°C.

3. The composition of Claim 1 wherein silicone gum (b) contains one or more M' units of the formula R⁴₃SiO_{1/2} and one or more additional units selected from amongst D' units of the formula R⁵₂SiO_{2/2}, T' units of the formula R⁶SiO_{3/2} and Q' units of the formula SiO_{4/2}, and mixtures thereof, wherein each R⁴, R⁵ and R⁶ is independently a hydroxyl radical or a monovalent hydrocarbon radical.

4. The composition of Claim 1 wherein silicon gum (b) is at least one of dimethiconol gum, dimethicone gum and polydimethysilicone gum.

5. The composition of Claim 1 wherein the weight ratio of silicone resin (a) to silicone gum (b) ranges from 0.4 to 6.

6. The composition of Claim 1 which is cationic surfactant-free.

7. The composition of Claim 1 further comprising a volatile organic solvent for the mixture of silicone resin (a) and silicone gum (b).

8. A personal care product which comprises the composition of any one of claims 1 to 5 and
a) volatile organic solvent for the mixture of silicone resin (a) and silicone gum (b)

9. The personal care product of Claim 8 wherein the personal care product is a cationic surfactant-free hair care product in which the mixture of silicone resin (a) and silicone gum (b) is dissolved in volatile organic solvent.

10. The composition of Claim 1 wherein the personal cared product is a lip gloss.

## Patentansprüche

1. Zusammensetzung, welche umfasst:
a) ein MT-Harz mit einer relativen Molekülmasse von über 10.000, das ein oder mehrere M-Einheiten der Formel R¹₃SiO_{1/3} und T-Einheiten der Formel R³SiO_{3/2} enthält, wobei das Verhältnis von M- zu T-Einheiten von 1:1 bis 1:7 reicht und der Erweichungspunkt des MT-Harzes von 50° bis 110° C reicht, wobei jedes R¹ und R³ unabhängig ein Hydroxylradikal oder ein monovalentes Kohlenwasserstoffradikal ist; und
b) Silikonkautschuk,
wobei die Mischung von MT-Harz (a) und Silikonkautschuk (b) nicht vernetzt ist und einen Erweichungspunkt von über 50°C und ein Elastizitätsmodul bei Umgebungstemperatur von weniger als 10⁶Pa aufweist.

2. Zusammensetzung nach Anspruch 1, wobei Silikonkautschuk (b) eine Viskosität von 0,3 bis 200.000 Pa·s (von 300 bis 200.000.000 Centipoise) bei 25°C aufweist.

3. Zusammensetzung nach Anspruch 1, wobei Silikonkautschuk (b) ein oder mehrere M'-Einheiten der Formel R⁴₃SiO_{1/2} und ein oder mehrere zusätzliche Einheiten gewählt aus D'-Einheiten der Formel R⁵₂SiO_{2/2}, T'-Einheiten der Formel R⁶SiO_{3/2} und Q'-Einheiten der Formel SiO_{4/2} und Mischungen derselben enthält, wobei jedes R⁴, R⁵ und R⁶ unabhängig ein Hydroxylradikal oder ein monovalentes Kohlenwasserstoffradikal ist.

4. Zusammensetzung nach Anspruch 1, wobei Silikonkautschuk (b) mindestens eines von Dimethiconolkautschuk, Dimethiconkautschuk und Polydimethysilikonkautschuk ist.

5. Zusammensetzung nach Anspruch 1, wobei das Gewichtsverhältnis von Silikonharz (a) zu Silikonkautschuk (b) von 0,4 bis 6 reicht.

6. Zusammensetzung nach Anspruch 1, welche kationisch tensidfrei ist.

7. Zusammensetzung nach Anspruch 1, welche weiterhin ein flüchtiges organisches Lösungsmittel für die Mischung aus Silikonharz (a) und Silikonkautschuk (b) umfasst.

8. Körperpflegeprodukt, welches die Zusammensetzung nach einem der Ansprüche 1 bis 5 und
a) flüchtiges organisches Lösungsmittel für die Mischung aus Silikonharz (a) und Silikonkautschuk (b) umfasst.

9. Körperpflegeprodukt nach Anspruch 8, wobei das Körperpflegeprodukt ein kationisches tensidfreies Haarpflegeprodukt ist, in dem die Mischung aus Silikonharz (a) und Silikonkautschuk (b) in flüchtigem organischen Lösungsmittel aufgelöst ist.

10. Zusammensetzung nach Anspruch 1, wobei das Körperpflegeprodukt ein Lipgloss ist.

## Revendications

1. Composition qui comprend :
a) une résine MT ayant une masse moléculaire supérieure à 10 000 et contenant un ou plusieurs motifs M de formule R¹₃SiO_{1/3} et des motifs T de formule R³SiO_{3/2} où le rapport de motifs M sur T varie de 1:1 à 1:7, et le point de ramollissement de la résine MT varie de 50 ° à 110 °C où chaque R¹ et R³ est indépendamment un radical hydroxyle ou un radical hydrocarbure monovalent ; et,
b) une gomme de silicone, où
le mélange de résine MT (a) et de gomme de silicone (b) est non réticulé et a un point de ramollissement supérieur à 50 °C et un module d'élasticité à température ambiante inférieur à 10⁶ Pa.

2. Composition selon la revendication 1 où
la gomme de silicone (b) possède une viscosité allant de 0,3 à 200 000 Pa·s (de 300 à 200 000 000 centipoises) à 25 °C.

3. Composition selon la revendication 1 où
la gomme de silicone (b) contient un ou plusieurs motifs M' de formule R⁴₃SiO_{1/2} et un ou plusieurs motifs supplémentaires choisis parmi des motifs D' de formule R⁵₂SiO_{2/2}, des motifs T' de formule R⁶SiO_{3/2} et des motifs Q' de formule SiO_{4/2}, et des mélanges de ceux-ci, où chaque R⁴, R⁵ et R⁶ est indépendamment un radical hydroxyle ou un radical hydrocarbure monovalent.

4. Composition selon la revendication 1 où
la gomme de silicone (b) est l'une au moins parmi de la gomme de diméthiconol, de la gomme de diméthicone et de la gomme de polydiméthysilicone

5. Composition selon la revendication 1 où
le rapport massique de la résine de silicone (a) sur la gomme de silicone (b) varie de 0,4 à 6.

6. Composition selon la revendication 1 qui est dépourvue de tensioactif cationique.

7. Composition selon la revendication 1 comprenant en outre un solvant organique volatil pour le mélange de résine de silicone (a) et de gomme de silicone (b).

8. Produit de soin personnel qui comprend la composition selon l'une quelconque des revendications 1 à 5 et
a) un solvant organique volatil pour le mélange de résine de silicone (a) et de gomme de silicone (b)

9. Produit de soin personnel selon la revendication 8 où
le produit de soin personnel est un produit de soin capillaire dépourvu de tensioactif cationique dans lequel le mélange de résine de silicone (a) et de gomme de silicone (b) est dissous dans un solvant organique volatil.

10. Composition selon la revendication 1 où le produit de soin personnel est un brillant pour les lèvres.
